(19) Europäisches Patentamt
European Patent Office
Office européen des brevets

(11) EP 2 502 978 A1

(12) **EUROPEAN PATENT APPLICATION**

(43) Date of publication:
**26.09.2012 Bulletin 2012/39**

(51) Int Cl.:
***C11B 9/00*** *(2006.01)* ***A61Q 13/00*** *(2006.01)*

(21) Application number: **12161085.1**

(22) Date of filing: **23.03.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.03.2011 US 201113071244**

(71) Applicant: **International Flavors & Fragrances Inc.**
**New York, NY 10019 (US)**

(72) Inventors:
- **Young, Timothy J**
  **Middletown, NJ New Jersey 07748 (US)**
- **Benaim, Carlos**
  **Bedford Hills, NY New York 10507 (US)**
- **Kerschner, Judith L**
  **Hawthorne, NJ New Jersey 07056 (US)**
- **Laudamiel, Christophe**
  **New York, NY New York 10011 (US)**

(74) Representative: **Lawrence, John**
**Barker Brettell LLP**
**100 Hagley Road**
**Edgbaston**
**Birmingham**
**B16 8QQ (GB)**

# (54) High performance fragrance formulation

(57) A method of formulating a high performance fragrance formulation by selecting an effective amount of fragrance ingredients selected from the group consisting of aroma chemicals and essential oils defined according to the following algorithm

$$7.393*\frac{P}{V}+\Psi V+\Delta*\frac{A}{V}=1$$

wherein P is equal to molecular polarizability, V is equal to molecular volume, A is equal to the molecular surface area. Ψ is a constant falling within the range {.0025-.0055} and Δ is a constant falling in the range of {-0.55 to -1.0} and the pair result in the above equality.

**Figure 1**

The data as graphed clearly indicates that Fragrance 2 is perceived significantly higher in intensity than Fragrance 1 at all three evaluations.

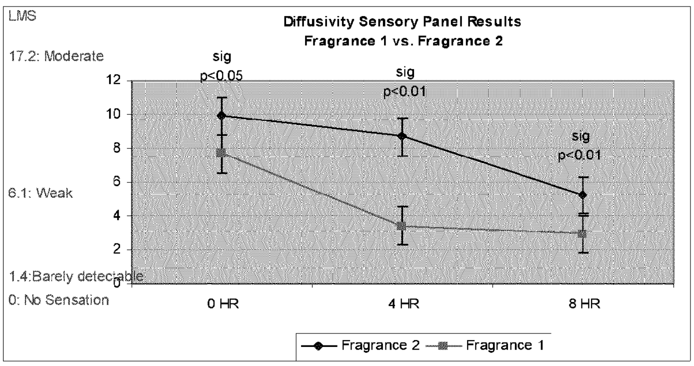


EP 2 502 978 A1

## Description

### Introduction

This patent application claims the benefit of priority from U.S. Patent Application Serial Number 13/071,244 filed March 24, 2011, the content of which is incorporated herein by reference in its entirety.

### Field of the Invention

The following invention is directed to creating a high performance fragrance formulation.

### Background of the Invention

In the early 1960's, Jean Carles developed a method to create fine fragrances by defining a fragrance as being made up of with ingredients designated as top, middle and base notes and with the fragrance formulation containing 25% top, 20% middle and 55% base notes (Series of publications in *Researches,* 1961,1962, 1963). Top notes are defined as volatile ingredients that lack tenacity on blotters or smelling strips and that provide strong performance at initial product application. Middle notes or "modifiers" have intermediate volatility and tenacity on blotters while the heavier base notes provide the long lasting component expected in every fragrance.

In practice, it is usually the case that many of the compounds considered "top" notes are fleeting, and disappear from the vapor around the wearer within the first five minutes of application. While these notes contribute a pleasurable moment for the user, they contribute little to the aroma that another observer would recognize as the signature consumers identify with of a particular fine fragrance. Therefore the aura the wearer projects over the first few hours is composed of compounds which are diffusive over time, and compounds which gradually decline in the vapor as the fragrance evolves. These compounds are distinguished in the present invention from the top and middle dichotomy, to simply one class known as 'push'.

One important performance parameter for fine fragrances is their ability to be noticed and appreciated in the "air" around the fragrance user. This parameter is typically referred to as fragrance diffusivity. An added benefit for most fine fragrances is their ability to be diffusive over time and therefore continue to provide performance during a typical use cycle for the fragrance. A high performing fine fragrance will combine this continued diffusivity benefit along with being designed to meet different olfactive and hedonic characteristics.

In efforts to increase the long-lasting, diffusive performance of fragrance additives such as fixatives, pro-fragrances and delivery systems have been disclosed in U.S. Patent No. 7,196,052, European Patent No. 0977548B2, and U.S. Patent No. 7,119,057. While these additives have been reported to offer fragrance performance benefits, they also can have issues depending on the fragrances product application. These issues can include negative interactions with the product and/or fragrance, inefficient mode of activation on skin, negative product sensory aspects and others.

We have surprisingly found that we can create long-lasting, high performing fragrances by designing these fragrances with a specific range of and combination of "diffusive" ingredients as defined by a diffusivity index without the addition of fixatives, profragrances or delivery vehicles.

By systematically using appropriate amounts of high performing diffusive and push ingredients and using the remainder of the formula to provide further olfactive and hedonic characteristics, one can create a high performing, long lasting diffusive fragrance that is hedonically pleasing to the consumer.

### Brief Description of the Drawings

Figure 1: The data as graphed clearly indicates that Fragrance 2 is perceived significantly higher in intensity than Fragrance 1 at all three evaluations

Figure 2: The data in the graph in Figure 2 illustrates predicted performance of fragrance on additional substrates and indicates that Fragrance 2 is perceived significantly higher in intensity than Fragrance 1 in this test.

### Summary of Invention

In one embodiment of the invention, a method of formulating a high performance fragrance formulation by selecting an effective amount of long-lasting, diffusive fragrance ingredients selected from the group consisting of aroma chemicals and essential oils defined according to the following algorithm

$$7.393 * \frac{P}{V} + \Psi V + \Delta * \frac{A}{V} = 1$$

wherein P is equal to molecular polarizability, V is equal to molecular volume, A is equal to the molecular surface area. $\Psi$ is a constant falling within the range {.0025-.0055} and $\Delta$ is a constant falling in the range of{ -0.55 to -1.0} and the pair result in the above equality. Compounds with a favorable push factor possess vapor pressures from about 0.1 to about 100 pa.

In another embodiment of the invention a method to formulate a high performance fragrance formulation is provided wherein at least 40 weight percent % of the formula by weight is comprised of fragrance ingredients where two constants ($\Psi$ and $\Delta$) can be found which are solutions to the equation and at least 5 weight percent % of formula by weight is comprised of compounds with a favorable push factor

In a further embodiment of the invention a method to formulate a high performance fragrance formulation is provided wherein about 50 weight percent % to about 90 weight percent % of all fragrance ingredients has constants ($\Psi$ and $\Delta$) which are a solution and about 5 to about 20weight percent % of the fragrance ingredients have a favorable push factor.

In yet a further embodiment of the invention, a method to formulate a high performance fragrance formulation is provided wherein the weighted average of the constant $\Delta$ is above -0.5, more preferably the weighted average of the constant $\Delta$ is above -0.6.

## Detailed Description of the Invention

According to the present invention, the effective diffusivity index was determined by correlating the long term effective performance of a compound applied on skin with the global molecular properties, specifically, molecular surface area, molecular volume, and polarizability/molar volume. The highly effective long lasting diffusive compounds are generally larger in size, with a relatively low surface area and high polarizability.

According to the present invention, molecular volume is defined as the molecular mass/molecular density. It is a measure of the volume occupied by a molecule (or scaled by moles, the volume occupied by a mole of molecules) condensed phase at room temperature and atmospheric pressure.

Molecular surface area is defined as the area of the Van der Waals surface area of a molecule. This is the area of an imaginary surface surrounding the molecule calculated where overlap between molecular orbitals of separate molecules in the condensed phase is of the lowest free energy.

Polarizability is defined as the capacity of a molecule to polarize in an electric field due to a displacement of mobile charges within the molecule. For a mole of molecules, these individual polarizabilities (p) sum to a molar polarizability (Np) value. Molecules with high polarizability tend to be unsaturated and sometimes aromatic and have functional groups which allow strong delocalization of electrons across pi systems between electron donor and acceptor pairs. These types of molecules tend to absorb strongly in the near UV and visible ranges.

Diffusivity is a time-space description of the transport of a property through a medium such as air. A highly desirable fragrance composition is one which can produce an experience not just at a point in time after application but over an extended period of several hours. This performance can be measured using purge and trap sampling techniques with subsequent GC and/or GC/MS analysis of the sample. This sampling technique allows fragrance concentrations to be quantified over the fragrance performance time-space continuum desired by consumers

The effective odor produced by the concert of compounds within a fragrance composition is described from a physical mode, and from a phenomenological mode.

As with any means to use physical or molecular properties of the fragrance composition, there are quantifiable uncertainties.

The structure of a compound has a profound effect on physical properties of the compound such as vapor pressure and water solubility, and on the neurobiological properties like odor character and odor threshold.

In one embodiment, the correlations of the global molecular and electronic properties with fragrance performance were explicitly established without appealing to secondary thermodynamic properties such as vapor pressure, water solubility, logP, Henry's law constant or boiling point.

Accordingly, the following algorithm has been developed to determine the proper combination of fragrance ingredients to create a high performance fragrance.

In one embodiment of the invention, a method of formulating a high performance fragrance formulation by selecting an effective amount of long-lasting, diffusive fragrance ingredients selected from the group consisting of aroma chemicals and essential oils defined according to the following algorithm:

$$7.393 * \frac{P}{V} + \Psi V + \Delta * \frac{A}{V} = 1$$

wherein P is equal to molecular polarizability, V is equal to molecular volume; A is equal to the molecular surface area. $\Psi$ is a constant falling within the range {.0025-.0055} and $\Delta$ is a constant falling in the range of {-0.55 to -1.0} and the pair result in the above equality. Compounds with a favorable push factor possess vapor pressures from about 0.1 to about 100 pa.

The mass or weight fractions of the compounds in the fragrance formula is designated by the algorithms to provide, in aggregate, those properties of a successful, highly performing benchmark fragrance that would be described as pleasantly diffusive.

One important performance parameter for fine fragrances is there ability to be noticed and appreciated in the "air" around the fragrance user. This parameter is typically referred to as fragrance diffusivity. An added benefit for most fine fragrances is their ability to be diffusive over time and therefore continue to provide performance during a typical use cycle for the fragrance. A high performing fine fragrance will combine this continued diffusivity benefit along with being designed to meet different olfactive/hedonic characteristics.

Fragrances for applied applications like body wash, shampoo, detergent, fabric conditioners, fabric refreshers, household cleaning products and the like are also contemplated by the present invention. In order to provide the highest fragrance impact from the fragrance deposited on the various substrates it is preferred that materials with a high performance be used. The substrates include but are not limited to skin, hair, textiles such as cloth and hard surfaces. This property must be balanced with the volatility as described above. Some of the principles mentioned above are disclosed in U.S. Patent No. 5,112,688.

In another embodiment of the invention, a method to formulate a high performance fragrance formulation is provided wherein at least 40% of the formula by weight is comprised of fragrance ingredients where two constants ($\Psi$ and $\Delta$) can be found which are solutions to the equation and at least 5 weight percent % of formula by weight is comprised of compounds with a favorable push factor.

In a further embodiment of the invention a method to formulate a high performance fragrance formulation is provided wherein about 50 weight percent to about 90 weight percent % of all fragrance ingredients have constants ($\Psi$ and $\Delta$) which are a solution and about 5 weight percent to about 20 weight percent % of the fragrance ingredients have a favorable push factor.

In yet a further embodiment of the invention, a method to formulate a high performance fragrance formulation is provided wherein the weighted average of the constant $\Delta$ is above -0.5, more preferably the weighted average of the constant $\Delta$ is above -0.6.

To determine high performing diffusive ingredients, different fragrance formulas were prepared and tested for their performance. Analytical measurements were used to identify the fragrance ingredients present in higher performing fragrance formulas. The effectiveness of individual fragrance ingredients was determined by combining the analytical and olfactive assessments.

Understanding the chemical and physical properties of fragrance ingredients that contribute to the desired long-lasting and diffusive properties of a final fragrance creation provides insight into the design of hedonically pleasing and high performing perfumes.

Others have defined "enduring" fragrances as containing very high percentages of ingredients with high boiling points and high partition coefficients, but it has been found that by using more complex ingredient parameters, fragrance ingredients can be ranked in their effectiveness as long-lasting and diffusive. By combining these long-lasting diffusive ingredients with "push" ingredients, the performance of the fragrance can be optimized over the entire use cycle.

"Push" is defined by the ability of particular compounds to appear in the vapor around the wearer at a significantly higher level than the mean ingredient (from among approximately 1000 tested). These compounds are characterized by a high level in the vapor directly after application and a very gradual decline over a few hours.

Some preferred high performing diffusive fragrance ingredients include but are not limited by the following: galaxolide, iso gamma super, tonalid, hedione, cedrol, ambroxan, benzyl salicylate, eugenol, vertofix, ethylene brassylate, helional, hexyl cinnamaldehyde, amyl salicylate, polysantol, damascene delta and methyl ionone alpha.

These analytical and olfactive assessments also defined the "push" ingredients and they include, but are not limited by the following ingredients: aldehyde AA, aldehyde C10, fenchyl acetate, terpineol, styryl acetate, methyl nonyl ketone, linalool, dihydromyrcenol, menthol, linalyl acetate, benzyl acetate, allyl heptanoate, citronellol, citronellyl nitrile, isobutyl benzoate and citronellal.

Understanding fine fragrance performance over time is critical and as previously described, a perfume must be designed to provide benefits over the entire use cycle of the product. To that end, analytical measurements were obtained at different time points and an understanding of which ingredients contributed most to the fragrances performance at different time points was gleaned from these measurements.

All U.S. patents and patent applications cited herein are incorporated by reference as if set forth herein in their entirety.

The following are provided as specific embodiments of the present invention. Other modifications of this invention will be readily apparent to those skilled in the art. Such modifications are understood to be within the scope of this invention. As used herein all percentages are weight percent unless otherwise noted, ppm is understood to stand for parts per million, L is understood to be liter, mL is understood to be milliliter, pa is understood to be pascal, g is understood to be gram, mol is understood to be mole, and mmHg be millimeters (mm) of mercury (Hg). IFF as used in the examples is understood to mean International Flavors & Fragrances Inc., New York, NY, USA.

Example I

Below are examples of three compounds with a favorable diffusivity index ($D_f$<-0.9), including calculations, and three compounds with unfavorable diffusivity indexes ($D_f$>-0.9):

| | **MOL VOL** angst^3 | **area/vol** ang^-1 | **polarizability/vol** | Δ | Ψ |
|---|---|---|---|---|---|
| GALAXOLIDE | 209.42 | 1.29 | 0.19 | 0.0030 | -0.8 |
| ISO GAMMA SUPER | 200.17 | 1.23 | 0.18 | 0.0033 | -0.8 |
| PATCHOULI ALC | 188.64 | 1.15 | 0.18 | 0.0031 | -0.8 |
| | | | | | |
| FENCHYL ACET | 162.98 | 1.30 | 0.16 | 0.0028 | -0.5 |
| CITRONELLYL NITRILE | 135.22 | 1.55 | 0.17 | 0.0027 | -0.4 |
| ISO BUTYL BENZOATE | 133.89 | 1.6 | 0.18 | 0.0025 | -0.4 |

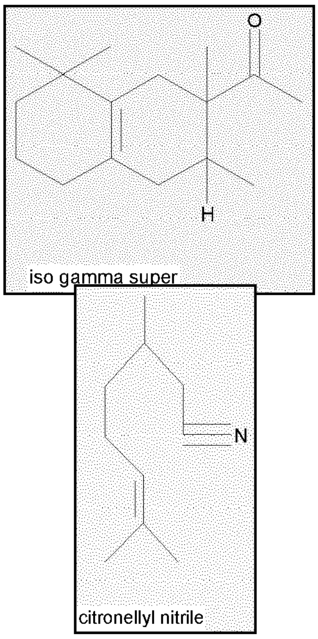

iso gamma super

citronellyl nitrile

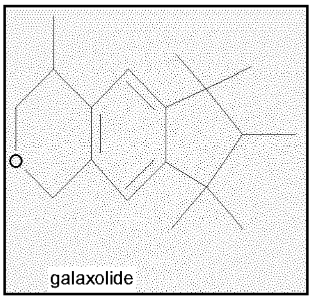

galaxolide

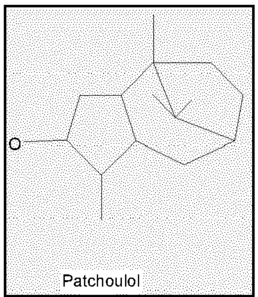

Patchoulol

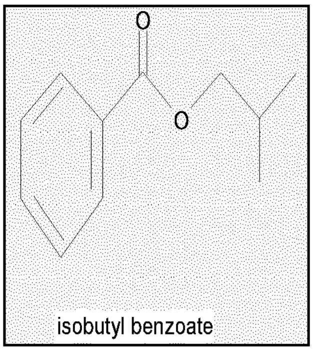

isobutyl benzoate

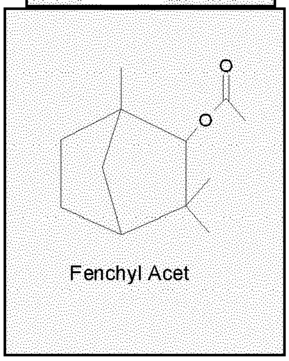

Fenchyl Acet

**Example II**

This example demonstrates two fragrances with very different performance. One fragrance was created to provide a weighted favorable long lasting, diffusive performance by providing a mixture containing >60 wt % diffusive

ingredients (Fragrance 2) and the other fragrance will have poor long lasting performance as this mixture only contains ~22 wt % diffusive ingredients. Measurements were made with TSAR Version 3 commercially available from Accelrys located in San Diego.

| Fragrance 1 | | | | Fragrance 2 | | | |
|---|---|---|---|---|---|---|---|
| Ingredient | Wt % | Δ | Ψ | Ingredient | Wt % | Δ | Ψ |
| Ald AA Triplal | 0.5 | -0.4 | 0.003 | Ald AA Triplal | 1.5 | -0.4 | 0.003 |
| Ald C-10 | 3.0 | -0.4 | 0.002 | Ald C-10 | 1.5 | -0.4 | 0.002 |
| Amber Core | 3.0 | -0.8 | 0.004 | Benzyl Salicylate | 7.0 | -0.8 | 0.003 |
| Amyl Acet | 3.0 | -0.4 | 0.004 | Cedrol | 4.0 | -0.8 | 0.0035 |
| Amyl Caproate | 3.0 | -0.4 | 0.003 | Eucalytol | 4.0 | -0.5 | 0.0035 |
| Amyl Salicylate | 10.0 | -0.6 | 0.0035 | Eugenol | 1.6 | -0.5 | 0.003 |
| Benzyl Acetate | 10.0 | -0.4 | 0.0035 | Iso Gamma Super | 10 | -0.8 | 0.003 |
| But Cinnamald | 5.0 | -0.6 | 0.003 | Galaxolide | 9.0 | -0.8 | 0.003 |
| Carvone D | 5.0 | -0.4 | 0.003 | Hexyl Cinnamald | 10 | -0.8 | 0.003 |
| Citronellyl Acet | 15.0 | -0.5 | 0.0035 | Limonene | 6.0 | -0.5 | 0.003 |
| Damascone Delta | 1.0 | -0.6 | 0.003 | Menthol | 1.8 | -0.5 | 0.0037 |
| Dihydro Myrcenol | 11.5 | -0.4 | 0.003 | Menthone | 2.6 | -0.4 | 0.003 |
| Eugenol | 1.0 | -0.5 | 0.0035 | Meth Ionone G | 3.0 | -0.6 | 0.0032 |
| Linalyl Acet | 10.0 | -0.5 | 0.003 | Patchoulol | 9.0 | -0.8 | 0.003 |
| Rosalva | 3.0 | -0.4 | 0.003 | Pinene Alpha | 4.0 | -0.5 | 0.0035 |
| T-hydro Myrcenol | 13.0 | -0.4 | 0.003 | Polysantol | 9.0 | -0.8 | 0.0035 |
| Vertofix | 3.0 | -0.8 | 0.003 | Terpinene Gamma | 2.0 | -0.5 | 0.003 |
| | | | | Terpinyl Acet | 4.0 | -0.5 | 0.0025 |
| | | | | Tonalid | 10 | -0.8 | 0.0035 |
| | Wt % of diffusive compounds 22.0 | | | | Wt % of diffusive compounds 61.0 | | |

A sensory test was performed to show the difference in long, lasting diffusive performance of these two fragrances. In this test a total of four sprays (total ca. 0.42-0.43g) is applied to four different areas of the wearer: one spray on each wrist and one on each side of the neck. Diffusivity evaluations were made at three time points: Initial impact, 4 hr & 8 hrs after product application. Each fragrance was applied to eight fragrance wearers and evaluated by eight judges trained in rating intensity of fragrances on labeled magnitude scale. The sensory judges evaluate the intensity of each fragrance from a distance of three feet while the fragrance wearers make ten passes walking briskly. Within each fragrance condition, the order in which the fragrance wearers walked past the judges is counterbalanced. Data analysis is performed by applying ANOVA to intensities of fragrances. Significance of the data is established at $p \leq 0.05$.

The data as graphed clearly indicates that Fragrance 2 is perceived significantly higher in intensity than Fragrance 1 at all three evaluations.

## Example III

This example demonstrates two fragrances with very different performance. One fragrance was created to provide a weighted favorable $\Sigma x_1 * \Delta_1$ <-0.6, average delta value ($WAD_f$) (Fragrance 2) which will provide good long lasting, diffusive properties and one fragrance with weight averaged delta which will have limited performance $\Sigma x_1 * \Delta_1$ >.-0.6. Measurements were made using TSAR Version 3 commercially available from Accelrys located in San Diego.

Fragrance 1

| Ingredient | wt % | D | wt%*delta/100 |
|---|---|---|---|
| Ald AA Triplal | 0.5 | -0.4 | -0.002 |
| Ald C-10 | 3.0 | -0.4 | -0.012 |
| Amber Core | 3.0 | -0.8 | -0.024 |
| Amyl Acet | 3.0 | -0.4 | -0.012 |
| Amyl Caproate | 3.0 | -0.4 | -0.012 |
| Amyl Salicylate | 10.0 | -0.6 | -0.06 |
| Benzyl Acetate | 10.0 | -0.4 | -0.04 |
| But Cinnamald | 5.0 | -0.6 | -0.03 |
| Carvone D | 5.0 | -0.4 | -0.02 |
| Citronellyl Acet | 15.0 | -0.5 | -0.075 |
| Damascone Delta | 1.0 | -0.6 | -0.006 |
| Dihydro Myrcenol | 11.5 | -0.4 | -0.046 |
| Eugenol | 1.0 | -0.5 | -0.005 |
| Linalyl Acet | 10.0 | -0.5 | -0.05 |
| Rosalva | 3.0 | -0.4 | -0.012 |
| T-hydro Myrcenol | 13.0 | -0.4 | -0.052 |
| Vertofix | 3.0 | -0.8 | -0.024 |
| Sum | 100.0 | | **-0.482** |

Fragrance 2

| Ingredient | wt % | D | wt%*delta/100 |
|---|---|---|---|
| Ald AA Triplal | 1.5 | -0.4 | -0.006 |
| Ald C-10 | 1.5 | -0.4 | -0.006 |
| Benzyl Salicylate | 7.0 | -0.6 | -0.042 |
| Cedrol | 4.0 | -0.8 | -0.032 |
| Eucalytol | 4.0 | -0.5 | -0.02 |
| Eugenol | 1.6 | -0.5 | -0.008 |
| Iso Gamma Super | 10.0 | -0.8 | -0.08 |
| Galaxolide | 9.0 | -0.8 | -0.072 |
| Hexyl Cinnamald | 10.0 | -0.8 | -0.08 |
| Limonene | 6.0 | -0.5 | -0.03 |
| Menthol | 1.8 | -0.5 | -0.009 |
| Menthone | 2.6 | -0.4 | -0.0104 |
| Meth Ionone G | 3.0 | -0.6 | -0.018 |
| Patchoulol | 9.0 | -0.8 | -0.072 |
| Pinene Alpha | 4.0 | -0.5 | -0.02 |
| Polysantol | 9.0 | -0.8 | -0.072 |
| Terpinene Gamma | 2.0 | -0.5 | -0.01 |
| Terpinyl Acet | 4.0 | -0.5 | -0.02 |
| Tonalid | 10.0 | -0.8 | -0.08 |
| Sum | 100.0 | | **-0.6874** |

**Example IV: Predicted Performance of Fragrances on Additional Substrates**

This example demonstrates that the predicted performance of the two fragrances from Example II is also replicated on additional substrates.

In this test, Fragrance 1 (predicted poor performer) and Fragrance 2 (predicted long lasting performer) were sprayed on cotton terry cloths and evaluated in a sensory test for their long-lasting diffusivity performance.

In this test, two cloths were sprayed with Fragrance 1 and two cloths were sprayed with Fragrance 2 (2 sprays on each cloth of ca. 0.21-0.22g).

The cloths were left in an open environment for twenty four (24) hours and then evaluated by a sensory panel of 14 judges trained in rating intensity of fragrances on a labeled magnitude scale. The judges were asked to evaluate the intensity of each fragrance, in duplication, at a distance of three feet from the cloth with a small fan blowing over it (to represent the diffusion of a fragrance from a person wearing clothing).

Within each fragrance condition, the order in which the fragrance wearers evaluated the four cloths was randomized. Data analysis is performed by applying ANOVA to the intensities of the fragrance. Significance of the data is established by $p \leq 0.05$.

The data in the graph in Figure 2 clearly indicates that Fragrance 2 is perceived significantly higher in intensity than Fragrance 1 in this test.

## Claims

**1.** A method of formulating a high performance fragrance formulation by selecting an effective amount of fragrance ingredients selected from the group consisting of aroma chemicals and essential oils defined according to the following algorithm:

$$7.393*\frac{P}{V}+\Psi V+\Delta*\frac{A}{V}=1$$

wherein P is equal to molecular polarizability; V is equal to molecular volume; A is equal to the molecular surface area; Ψ is a constant in the range {.0025-.0055} and Δ is a constant in the range of{-0.55 to -1.0}.

**2.** The method of claim 1 wherein at least 40% of the formula by weight is comprised of fragrance ingredients where Ψ is a constant in the range {.0025-.0055} and Δ is a constant in the range of{ -0.55 to -1.0}.

**3.** The method of claim 2 wherein at least 5% of the formula is comprised of compounds with a favorable push factor.

**4.** The method of claim 1 or of claim 2 or of claim 3 wherein from about 50 to about 90% of the selected fragrance ingredients have Ψ falling within the range {.0025-.0055} and Δ falling in the range of{ -0.55 to -1.0}.

**5.** The method of claim 4 wherein about 5% to about 20% of the fragrance ingredients have a favorable push factor.

**6.** The method of claim 4 or claim 5 wherein the fragrance ingredients with a favorable push factor are selected from the group consisting aldehyde AA, aldehyde C10, fenchyl acetate, terpineol, styryl acetate, methyl nonyl ketone, linalool, dihydromyrcenol, menthol, linalyl acetate, benzyl acetate, allyl heptanoate, citronellol, citronellyl nitrile, isobutyl benzoate and citronellal.

**7.** The method of claim 1 or of any preceding claim, wherein the weighted average of the constant Δ is greater than -0.6.

**8.** A high performance fragrance formulation prepared according to the method of claim 1 or of any of claims 2 to 7.

**9.** A high performance fragrance formulation prepared according to the method of claim 1 wherein at least 40% of the formula by weight is comprised of fragrance ingredients where Ψ is a constant within the range {.0025-.0055} and Δ is a constant in the range of{ -0.55 to -1.0}.

**10.** The high performance fragrance formulation of Claim 8 or of claim 9 wherein at least 5% of the formula is comprised of compounds with a favorable push factor.

**11.** The high performance fragrance formulation 10 wherein the fragrance ingredients with a favorable push factor are selected from the group consisting aldehyde AA, aldehyde C 10, fenchyl acetate, terpineol, styryl acetate, methyl nonyl ketone, linalool, dihydromyrcenol, menthol, linalyl acetate, benzyl acetate, allyl heptanoate, citronellol, citronellyl nitrile, isobutyl benzoate and citronellal.

**12.** A high performance fragrance formulation prepared according to the method of claim 1 wherein at least 50 to about 90% of the formula by weight is comprised of fragrance ingredients where Ψ is a constant within the range {.0025-.0055} and Δ is a constant in the range of{ -0.55 to -1.0}.

**13.** The high performance fragrance formulation of Claim 12 wherein about 5% to about 20% of the fragrance ingredients have a favorable push factor.

**14.** The high performance fragrance formulation of claim 12 or of claim 13 wherein the fragrance ingredients with a favorable push factor are selected from the group consisting aldehyde AA, aldehyde C10, fenchyl acetate, terpineol, styryl acetate, methyl nonyl ketone, linalool, dihydromyrcenol, menthol, linalyl acetate, benzyl acetate, allyl heptanoate, citronellol, citronellyl nitrile, isobutyl benzoate and citronellal.

**Figure 1**

The data as graphed clearly indicates that Fragrance 2 is perceived significantly higher in intensity than Fragrance 1 at all three evaluations.

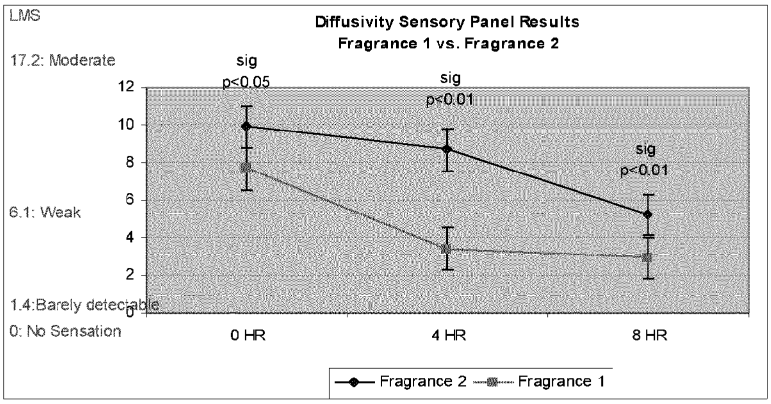


**Figure 2**

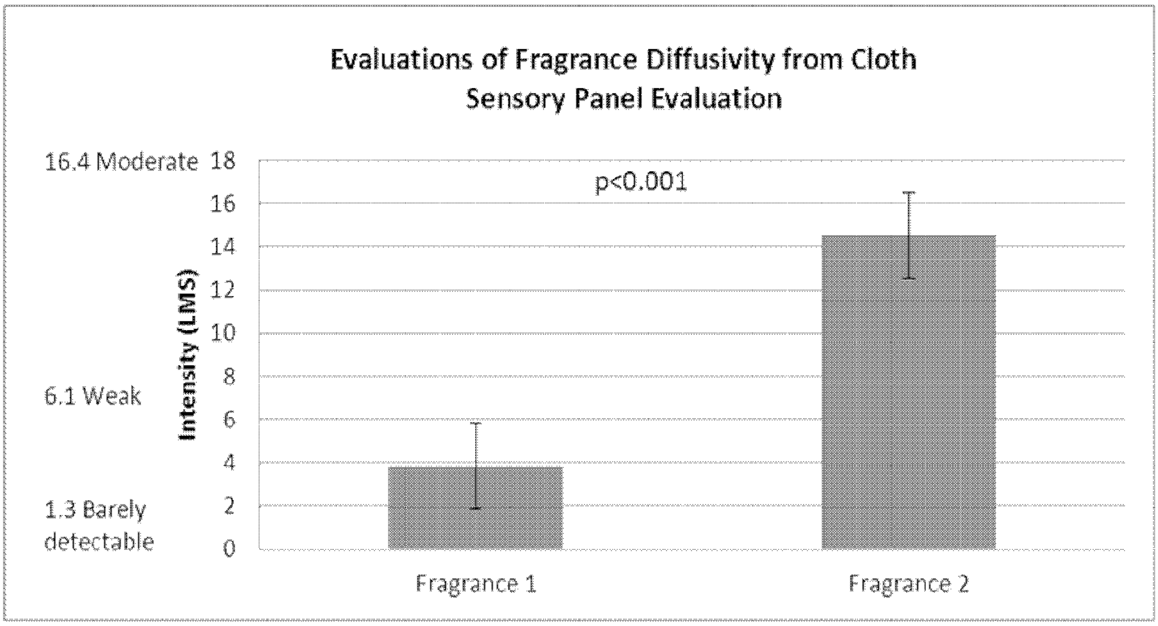

Europäisches Patentamt
European Patent Office
Office européen des brevets

# EUROPEAN SEARCH REPORT

Application Number
EP 12 16 1085

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 820 842 A1 (TAKASAGO PERFUMERY CO LTD) 22 August 2007 (2007-08-22)<br>* paragraphs [0060], [0068], [0083] *<br>----- | 1-14 | INV.<br>C11B9/00<br>A61Q13/00 |
| X | US 2007/099804 A1 (FADEL ADDI ET AL) 3 May 2007 (2007-05-03)<br>* table 6 *<br>----- | 1-14 | |
| X | US 2010/168253 A1 (SHOJI KEN ET AL) 1 July 2010 (2010-07-01)<br>* claims; table 11 *<br>----- | 1-14 | |
| X | EP 0 409 089 A2 (KAO CORP) 23 January 1991 (1991-01-23)<br>* claims; example C3 *<br>----- | 1-14 | |
| X | US 4 139 650 A (LIGHT KENNETH K ET AL) 13 February 1979 (1979-02-13)<br>* examples XX,XXXI *<br>----- | 1-14 | |
| A | WO 2010/058382 A1 (OREAL) 27 May 2010 (2010-05-27)<br>* claims *<br>----- | 1-14 | TECHNICAL FIELDS SEARCHED (IPC)<br>C11B<br>A61Q |
| A | GB 1 253 678 A (RANDEBROCK RUDOLF) 17 November 1971 (1971-11-17)<br>* claims *<br>----- | 1-14 | |

The present search report has been drawn up for all claims

1

| Place of search | Date of completion of the search | Examiner |
|---|---|---|
| Munich | 27 June 2012 | Hillebrecht, Dieter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 16 1085

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2012

| Patent document cited in search report | Publication date | Patent family member(s) | Publication date |
|---|---|---|---|
| EP 1820842 A1 | 22-08-2007 | CA 2587666 A1 | 18-05-2006 |
| | | EP 1820842 A1 | 22-08-2007 |
| | | JP 4808633 B2 | 02-11-2011 |
| | | US 2009143485 A1 | 04-06-2009 |
| | | US 2010305216 A1 | 02-12-2010 |
| | | US 2011313201 A1 | 22-12-2011 |
| | | WO 2006051597 A1 | 18-05-2006 |
| US 2007099804 A1 | 03-05-2007 | NONE | |
| US 2010168253 A1 | 01-07-2010 | CN 102329692 A | 25-01-2012 |
| | | EP 1921130 A1 | 14-05-2008 |
| | | EP 2143782 A1 | 13-01-2010 |
| | | ES 2375434 T3 | 29-02-2012 |
| | | KR 20080038156 A | 02-05-2008 |
| | | US 2010168253 A1 | 01-07-2010 |
| | | WO 2007015481 A1 | 08-02-2007 |
| EP 0409089 A2 | 23-01-1991 | DE 69015034 D1 | 26-01-1995 |
| | | DE 69015034 T2 | 24-05-1995 |
| | | EP 0409089 A2 | 23-01-1991 |
| | | JP 2562205 B2 | 11-12-1996 |
| | | JP 3052834 A | 07-03-1991 |
| US 4139650 A | 13-02-1979 | DE 2510621 A1 | 22-01-1976 |
| | | FR 2277064 A1 | 30-01-1976 |
| | | GB 1445068 A | 04-08-1976 |
| | | JP 51034140 A | 23-03-1976 |
| | | NL 7507788 A | 06-01-1976 |
| | | US 4139650 A | 13-02-1979 |
| WO 2010058382 A1 | 27-05-2010 | FR 2939000 A1 | 28-05-2010 |
| | | WO 2010058382 A1 | 27-05-2010 |
| GB 1253678 A | 17-11-1971 | CH 502432 A | 31-01-1971 |
| | | FR 1602132 A | 12-10-1970 |
| | | GB 1253678 A | 17-11-1971 |
| | | NL 6816527 A | 23-05-1969 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 07124411 A
- US 7196052 B
- EP 0977548 B2
- US 7119057 B
- US 5112688 A